(19) Europäisches Patentamt
European Patent Office
Office européen des brevets

(11) Veröffentlichungsnummer: **0 457 199 A1**

(12) **EUROPÄISCHE PATENTANMELDUNG**

(21) Anmeldenummer: **91107566.1**

(22) Anmeldetag: **09.05.91**

(51) Int. Cl.5: **C07D 417/04**, A61K 31/54

(30) Priorität: **13.05.90 DE 4015236**

(43) Veröffentlichungstag der Anmeldung: **21.11.91 Patentblatt 91/47**

(84) Benannte Vertragsstaaten: **AT BE CH DE DK ES FR GB GR IT LI NL SE**

(71) Anmelder: **CASSELLA Aktiengesellschaft**
**Hanauer Landstrasse 526**
**W-6000 Frankfurt am Main 60(DE)**

(72) Erfinder: **Schönafinger, Karl, Dr.**
**Holunderweg 8**
**W-8755 Alzenau(DE)**
Erfinder: **Beyerle, Rudi, Dr.**
**An der Pfaffenmauer 44**
**W-6000 Frankfurt 60(DE)**
Erfinder: **Bohn, Helmut, Dr.**
**Kranzbergring 11**
**W-6369 Schöneck(DE)**
Erfinder: **Just, Melitta, Dr.**
**Theodor-Heuss-Strasse 80**
**W-6070 Langen(DE)**

(74) Vertreter: **Urbach, Hans-Georg, Dr. et al**
**CASSELLA AKTIENGESELLSCHAFT**
**Patentabteilung Hanauer Landstrasse 526**
**W-6000 Frankfurt am Main 60(DE)**

(54) **Substituierte 3-Aminosydnonimine, Verfahren zu ihrer Herstellung und ihre Verwendung.**

(57) Substituierte 3-Aminosydnonimine der Formel I

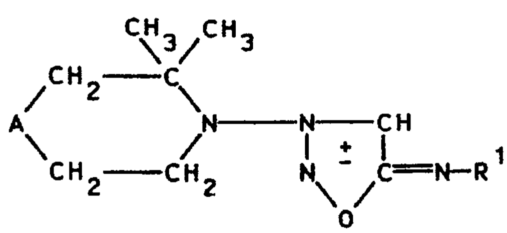

(I)

und ihre pharmakologisch annehmbaren Säureadditionssalze, worin
A den Rest -SO-,
$R^1$ den Rest $-COR^2$ oder Wasserstoff,
$R^2$ z.B. ($C_1$ bis $C_4$)Alkyl bedeuten, besitzen wertvolle pharmakologische Eigenschaften und können dadurch hergestellt werden, daß eine Verbindung der Formel I, worin A = -S- bedeutet, mit einer Sauerstoff abgebenden Substanz umgesetzt wird.

EP 0 457 199 A1

Die Erfindung betrifft pharmakologisch wirksame substituierte 3-Aminosydnonimine der allgemeinen Formel I

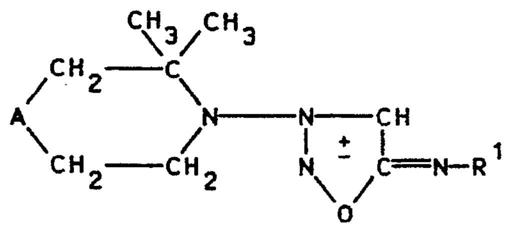

(I)

und ihre pharmakologisch annehmbaren Säureadditionssalze, worin
A den Rest -SO-,
$R^1$ den Rest -CO-$R^2$ oder Wasserstoff,
$R^2$ ($C_1$ bis $C_4$)Alkyl, ($C_1$ bis $C_4$)Alkoxy-($C_1$ bis $C_4$)alkyl, ($C_1$ bis $C_4$)Alkoxy, ($C_5$ bis $C_7$)Cycloalkyl, Phenyl, einen mit 1 bis 3 Halogenatomen und/oder 1 bis 3 Alkylresten mit 1 bis 4 C-Atomen und/oder 1 bis 3 Alkoxyresten mit 1 bis 4 C-Atomen mono-, di- oder trisubstituierten Phenylrest bedeuten.

Die Erfindung betrifft ferner Verfahren zur Herstellung der erfindungsgemäßen Verbindungen und ihre Verwendung.

Alkylreste, Alkoxyalkylreste und Alkoxyreste können geradkettig oder verzweigt sein. Dies gilt auch, wenn sie als Substituenten für Phenyl auftreten.

($C_1$ bis $C_4$)Alkylreste können sein: Methyl, Ethyl, n-Propyl, i-Propyl, n-Butyl, i-Butyl, tert.-Butyl, sec.-Butyl. ($C_1$ bis $C_4$)Alkoxyreste können z.B. sein: Methoxy, Ethoxy, n-Propoxy, i-Propoxy, n-Butoxy, i-Butoxy, sec.-Butoxy, tert.-Butoxy.

($C_1$ bis $C_4$)Alkoxy-($C_1$ bis $C_4$)alkylreste können z.B. sein: Methoxy-methyl, Ethoxy-methyl, Propoxy-methyl, Butoxy-methyl, i-Butoxy-methyl, 2-Methoxy-ethyl, 3-Methoxy-propyl, 4-Methoxy-butyl, 2-Ethoxy-ethyl, 3-Propoxy-propyl, 4-Propoxy-butyl, 2-Butoxy-ethyl, 3-Butoxy-propyl, 4-Butoxy-butyl, 3-i-Propoxy-propyl, 4-i-Propoxy-butyl, 2-Ethoxy-ethyl, 2-Propoxy-ethyl, 2-i-Propoxy-ethyl, 2-Butoxy-ethyl, 2-i-Butoxy-ethyl, 1-Methoxy-ethyl, 1-Ethoxy-ethyl, 1-Propoxy-ethyl, 2-Methoxy-propyl, 2-Ethoxy-propyl, 2-Propoxy-propyl, 2-i-Propoxy-propyl, 3-Methoxy-butyl, 3-Ethoxy-butyl, 3-Propoxy-butyl, 3-i-Propoxy-butyl, 3-Butoxy-butyl, 3-i-Butoxy-butyl, 3-tert.-Butoxy-butyl, 2-Methoxy-butyl, 2-Ethoxy-butyl, 2-Propoxy-butyl, 2-i-Propoxy-butyl, 2-Butoxy-butyl, 2-i-Butoxy-butyl.

Von den ($C_5$ bis $C_7$)Cycloalkylresten sind Cyclopentyl und Cyclohexyl bevorzugt.

Als Halogenatome für den substituierten Phenylrest kommen Fluor, Chlor, Brom und/oder lod in Betracht, von denen Brom und Chlor bevorzugt sind.

$R^2$ bedeutet vorzugsweise ($C_1$ bis $C_4$)Alkyl, ($C_1$ bis $C_4$)Alkoxy, Phenyl, oder durch Chlor, Methyl oder Methoxy monosubstituiertes Phenyl, wobei das monosubstituierte Phenyl vorzugsweise in 4- oder 2-Stellung substituiert ist.

Für $R^1$ ist Wasserstoff bevorzugt. Bevorzugte Verbindungen sind: N-p-Anisoyl-3-(3,3-dimethyl-perhydro-1-oxo-1,4-thiazin-4-yl)-sydnonimin, N-Ethoxycarbonyl-3-(3,3-dimethyl-perhydro-1-oxo-1,4-thiazin-4-yl)-sydnonimin und N-Pivaloyl-3-(3,3-dimethyl-perhydro-1-oxo-1,4-thiazin-4-yl)-sydnonimin sowie die pharmakologisch annehmbaren Säureadditionssalze der vorgenannten Verbindungen, insbesondere ihre Hydrochloride, sowie die pharmakologisch annehmbaren Säureadditionssalze, insbesondere das Hydrochlorid der Verbindung 3-(3,3-Dimethyl-perhydro-1-oxo-1,4-thiazin-4-yl)-sydnonimin.

Eine Verbindung der allgemeinen Formel I kann dadurch hergestellt werden, daß eine Verbindung der allgemeinen Formel II

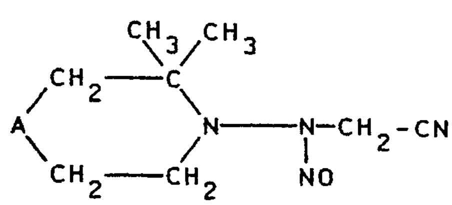

(II)

worin A die bereits genannte Bedeutung besitzt, zu einer Verbindung der allgemeinen Formel Ia

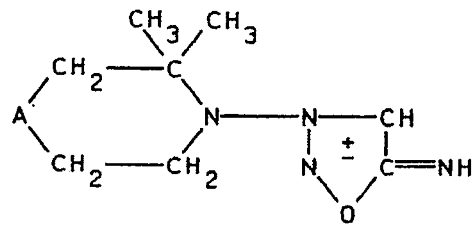

(Ia)

cyclisiert und die erhaltene Verbindung als Säureadditionssalz isoliert wird, oder daß für den Fall, daß eine Verbindung der Formel I mit $R^1$ = $-COR^2$ hergestellt werden soll, die Verbindung Ia oder ein Säureadditionssalz davon mit einem Acylierungsmittel, das den Rest $-COR^2$ einführt, acyliert wird und die so erhaltene Verbindung isoliert oder gegebenenfalls in ein pharmakologisch annehmbares Säureadditionssalz überführt, und dieses isoliert wird.

Die Cyclisierung der Verbindungen II zu den Verbindungen Ia kann in einem geeigneten organischen oder anorganischen Lösungs-, Dispergier- oder Verdünnungsmittel unter Zusatz eines Cyclisierungsmittels, normalerweise bei Temperaturen von -10 bis 40°C, insbesondere 0 bis 40°C, vorzugsweise bei 0 bis 20°C, durchgeführt werden.

Als Cyclisierungsmittel sind solche geeignet, die in wäßriger Lösung einen pH-Wert unter 3 einstellen, also z.B. Mineralsäuren, wie Schwefel-, Salpeter- oder Phosphorsäure, vorzugsweise Chlorwasserstoff, aber auch starke organische Säuren, wie Trifluoressigsäure. Die Cyclisierung wird normalerweise unter Eiskühlung durchgeführt.

Von dem Cyclisierungsmittel kommen z.B. bezogen auf 1 mol der Verbindung der Formel II 0,1 bis 10 mol, vorzugsweise 1 bis 5 mol, zur Anwendung. Das Cyclisierungsmittel wird normalerweise im Überschuß eingesetzt. Besonders bequem ist die Verwendung von Chlorwasserstoff als Cyclisierungsmittel, der normalerweise bis zur Sättigung in den Reaktionsansatz eingeleitet wird. Bei der Cyclisierung wird normalerweise das entsprechende Säureadditionssalz der Verbindung Ia erhalten.

Geeignete Lösungs-, Dispergier- oder Verdünnungsmittel sind z.B.: Alkohole, beispielsweise solche mit 1 bis 8 C-Atomen, insbesondere solche mit 1 bis 6 C-Atomen, vorzugsweise solche mit 1 bis 4 C-Atomen, wie z.B. Methanol, Ethanol, i- und n-Propanol, i-, sec- und tert-Butanol, n-, i-, sec-, tert-Pentanol, n-Hexanol, 2-Ethylbutanol, 2-Ethylhexanol, Isooctylalkohol, Cyclopentanol, Cyclohexanol, Methylcyclohexanol (Gemisch), Benzylalkohol; Ether, insbesondere solche mit 2 bis 8 C-Atomen im Molekül, wie z.B. Diethylether, Methyl-ethylether, Di-n-propyl-ether, Di-isopropyl-ether, Methyl-n-butylether, Methyl-tert-butylether, Ethyl-propyl-ether, Di-butylether, Tetrahydrofuran, 1,4-Dioxan, 1,2-Dimethoxyethan, Bis-β-methoxyethylether; Oligoethylen-glykol-dimethylether, wie z.B Tetraglyme oder Pentaglyme; Carbonsäurealkylester, insbesondere solche mit 2 bis 10 C-Atomen im Molekül, wie z.B. Ameisensäure-methyl-, -ethyl-, -butyl-oder -isobutyl-ester, Essigsäure-methyl-, -ethyl-, -propyl-, isopropyl-, -butyl-, -isobutyl- oder -secbutyl-, -amyl-, -isoamyl-, -hexyl-, -cyclohexyl- oder -benzyl-ester, Propionsäure-methyl-, -ethyl- oder -butylester; Ketone, insbesondere solche mit 3 bis 10 C-Atomen im Molekül, wie z.B. Aceton, Methylethylketon, Methyl-n-propylketon, Diethylketon, 2-Hexanon, 3-Hexanon, Di-n-propylketon, Di-iso-propylketon, Di-iso-butylketon, Cyclopentanon, Cyclohexanon, Methylcyclohexanon, Di-methylcyclohexanon, Benzophenon, Acetophenon; aliphatische Kohlenwasserstoffe, wie z.B. Hexan, Heptan, niedrig- und hochsiedende Petrolether, Spezialbenzine und Testbenzin; cycloaliphatische Kohlenwasserstoffe, wie z.B. Cyclopentan, Cyclohexan, Methylcyclohexan, Tetralin, Decalin; aromatische Kohlenwasserstoffe, wie z.B. Benzol, Toluol, o-, m- und p-Xylol, Ethylbenzol; halogenierte aliphatische oder aromatische Kohlenwasserstoffe, wie z.B. Methylenchlorid, Chloroform, Tetrachlorkohlenstoff, 1,2-Dichlorethan, Chlorbenzol, Dichlorbenzol; Hexamethylphosphorsäuretriamid; Sulfoxide, wie z.B. Dimethyl-sulfoxid; Tetramethylensulfon; Wasser. Auch Gemische verschiedener Lösungs- oder Dispergiermittel können verwendet werden, beispielsweise Wasser-Methanol oder vorzugsweise Essigsäureethylester-Methanol.

Die Acylierung der Verbindung der Formel Ia, die auch in Form eines Säureadditionssalzes vorliegen kann, zur Einführung des Restes $R^1$ = $-COR^2$ kann in an sich bekannter Weise mit einem geeigneten Acylierungsmittel der Formel III durchgeführt werden

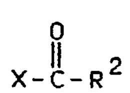

(III)

worin X ein nukleophil abspaltbarer Rest darstellt.

In der Formel III bedeutet X z.B. insbesondere Halogen, vorzugsweise -Cl oder -Br; -OH; -O-Alkyl, insbesondere mit 1 bis 5 C-Atomen; -O-Aryl, wobei der Arylrest insbesondere ein Phenylrest ist, der auch mit Alkyl, insbesondere Methyl, und/oder Nitro ein- oder mehrfach substituiert sein kann und beispielsweise ein Tolyl-, Dinitrophenyl- oder Nitrophenyl-Rest ist; -O-CO-$R^2$; -O-CO-O-Alkyl, insbesondere mit 1 bis 5 C-Atomen im Alkylrest, oder den über ein N-Atom gebundenen Rest eines Azols oder Benzazols mit mindestens 2 N-Atomen im quasiaromatischen Fünfring.

Die Acylierung wird zweckmäßigerweise in flüssiger oder flüssiger disperser Phase in Anwesenheit eines inerten Lösungs-, Dispergier- oder Verdünnungsmittels oder in einem Überschuß des Acylierungsmittels, zweckmäßigerweise unter Rühren, durchgeführt.

Bei der Acylierung beträgt das Molverhältnis zwischen der Verbindung der Formel Ia und dem Acylierungsmittel der Formel III theoretisch 1 : 1. Das Acylierungsmittel kann jedoch auch im Über- oder Unterschuß eingesetzt werden. Zweckmäßigerweise wird das Acylierungsmittel der Formel III im Überschuß eingesetzt. Überschüsse von bis zu 30 mol% sind in der Regel ausreichend, d.h. das Molverhältnis zwischen der Verbindung der Formel Ia und dem Acylierungsmittel der Formel III beträgt normalerweise 1 : (1 bis 1,3), vorzugsweise 1: (1 bis 1,2). Falls bei der Acylierungsreaktion eine Säure abgespalten wird, ist der Zusatz eines Säurefängers, wie z.B. eines Alkalimetallhydroxids, wie z.B. Natrium-, Kalium- oder Lithiumhydroxid, eines tertiären organischen Amins, wie z.B. Pyridin oder Triethylamin, eines Alkalimetallcarbonats oder Alkalimetallbicarbonats, wie z.B. Soda oder Natriumbicarbonat, oder eines Alkalimetallsalzes einer schwachen organischen Säure, wie z.B. Natriumacetat, zweckmäßig. Bei der Acylierungsreaktion können auch geeignete Katalysatoren, wie z.B. 4-Dimethylaminopyridin, zugesetzt werden.

Die Acylierung kann prinzipiell bei Temperaturen zwischen -10°C und dem Siedepunkt des verwendeten Lösungs-, Dispergier- oder Verdünnungsmittels erfolgen. In vielen Fällen wird die Umsetzung bei 0 bis 50°C, insbesondere bei 0 bis 30°C und vorzugsweise bei Raumtemperatur, durchgeführt.

Die Verbindungen der Formel III sind Acylierungsmittel und stellen somit z.B. dar: für X = Halogen: Säurehalogenide bzw. Halogenameisensäureester, von denen Säurechloride und Chlorameisensäureester bevorzugt sind; für -OH: Carbonsäuren; für -O-Alkyl und -O-Aryl: Ester, von denen die Tolyl-, 2,4-Dinitro- oder 4-Nitrophenylester bevorzugt sind; für -O-CO-R: Anhydride; für -O-CO-O-Alkyl; gemischte Carbonsäure-Kohlensäure-Anhydride; oder heterocyclische Amide oder Azolide, insbesondere von N,N'-Carbonyldiazolen, wie z.B. N,N'-Carbonyldiimidazol, 2,2'-Carbonyl-ditriazol(1,2,3), 1,1'-Carbonylditriazol-(1,2,4), N,N'-Carbonyl-dipyrazol, 2,2'-Carbonylditriazol (vgl. z.B. H.A. Staab, M. Lücking und F.H. Dürr, Chem. Ber. 95, (1962), 1275 ff, H. A. Staab und A. Mannschreck, Chem. Ber. 95, (1962), 1284 ff.; H.A. Staab und W. Rohr, "Synthesen mit heterocyclischen Amiden (Azoliden)" in "Neuere Methoden der Präparativen Organischen Chemie", Band V, Verlag Chemie, 1967, S. 53 ff., insbesondere S. 65 bis 69). Die Acylierungsmittel der Formel III können nach an sich bekannten Verfahren hergestellt werden.

Bei der Verwendung einer Carbonsäure als Acylierungsmittel ist der Zusatz eines Aktivierungsmittels zweckmäßig, das die Aufgabe hat, das Acylierungspotential der Carbonsäure zu erhöhen oder zu aktivieren bzw. die Carbonsäure in situ oder vorzugsweise kurz vor der Umsetzung mit der Verbindung der Formel Ia in ein reaktives Carbonsäurederivat der Formel III zu überführen. Als derartige Aktivierungsmittel sind z.B. geeignet: N,N'-disubstituierte Carbodiimide, insbesondere wenn sie mindestens einen sekundären oder tertiären Alkylrest enthalten, wie z.B. Diisopropyl-, Dicyclohexyl- oder N-Methyl-N'-tert.butylcarbodiimid (vgl. Methodicum Chimicum, Verlag G.Thieme, Stuttgart, Bd.6, (1974), S.682/683, und Houben-Weyl, Methoden der Org. Chemie, Bd.8, (1952), S. 521/522); Kohlensäurederivate, wie z.B. Phosgen, Chlorameisensäureester, insbesondere mit 1 bis 5 C-Atomen im Alkylrest (vgl. z.B. Tetrahedron Letters 24 (1983), 3365 bis 3368); Kohlensäureester, wie z.B. N,N'-Disuccinimido-carbonat, Diphthalimido-carbonat, 1,1'-(Carbonyldioxy)-dibenzo-triazol oder Di-2-pyridyl-carbonat (vgl. z.B. Tetrahedron Letters, Vol.25, No.43, 4943-4946), gegebenenfalls in Anwesenheit geeigneter Katalysatoren, wie z.B. 4-Dimethylaminopyridin. Ferner sind als Aktivierungsmittel N,N'-Carbonyldiazole, wie z.B. N,N'-Carbonyl-diimidazol, 2,2'-Carbonylditriazol(1,2,3), 1,1'-Carbonyl-ditriazol-(1,2,4), N,N'-Carbonyl-dipyrazol, 2,2'-Carbonyl-ditetrazol, N,N'-Carbonyl-benzimidazol oder N,N'-Carbonylbenztriazol, geeignet (vgl. z.B. H.A. Staab, M. Lücking und F.H. Dürr, loc. cit; H.A. Staab und A. Mannschreck loc. cit.; H.A. Staab und W. Rohr loc. cit). Als N,N'-Carbonyldiazol wird häufig das käufliche N,N'-Carbonyl-diimidazol verwendet. Die anderen N,N'-Carbonylazole sind aber aus dem jeweiligen Azol und Phosgen ebenfalls leicht zugänglich.

Als Aktivierungsmittel für Carbonsäuren sind ferner geeignet: Derivate der Oxalsäure, wie z.B. Oxalylchlorid (vgl. z.B. GB-PS 2 139 225) oder N,N'-Oxalyl-diazole wie z.B. 1,1'-Oxalyldi-imidazol, 1,1'-Oxalyldi-1,2,4-triazol und 1,1'-Oxalyldi-1,2,3,4-tetrazol (vgl. z.B. Shizuaka Murata, Bull.Chem. Soc.Jap. 57, 3597-3598 (1984)); Methylethylphosphinsäureanhydrid (vgl. z.B. DE-OS 31 01 427); Diphosphortetrajodid (Chem. Lett. 1983, 449); Dialkyldisulfit (Indian J. Chem. 21, 259 (1982)); oder andere reaktive Agentien.

Geeignete Lösungs-, Dispergier- oder Verdünnungsmittel sind z.B. solche, die für die Durchführung der Cyclisierung angegeben worden sind, darüber hinaus auch z.B. Pyridin und Amide, wie z.B. Dimethylformamid. Neben Wasser werden für die Acylierung polare organische Lösungsmittel, wie Dimethylformamid, Dimethylsulfoxid oder Pyridin bevorzugt. Auch Lösungsmittelgemische, wie z.B. ein Gemisch aus Wasser und Methylenchlorid, sind geeignet.

Die substituierten 3-Amino-sydnonimine der allgemeinen Formel I können mit anorganischen oder organischen Säuren Säureadditionssalze bilden. Zur Bildung derartiger Säureadditionssalze sind anorganische oder organische Säuren geeignet. Für die Bildung pharmakologisch annehmbarer Säureadditionssalze sind geeignete Säuren beispielsweise: Chlorwasserstoff, Bromwasserstoff, Naphthalindisulfonsäuren, insbesondere Naphthalindisulfonsäure(1,5), Phosphor-, Salpeter-, Schwefel-, Oxal-, Milch-, Wein-, Essig-, Salicyl-, Benzoe-, Ameisen-, Propion-, Pivalin-, Diethylessig-, Malon-, Bernstein-, Pimelin-, Fumar-, Malein-, Apfel-, Sulfamin-, Phenylpropion-, Glucon-, Ascorbin-, Isonicotin-, Methansulfon-, p-Toluolsulfon-, Zitronen- oder Adipinsäure. Die Säureadditionssalze können wie üblich durch Vereinigung der Komponenten, zweckmäßigerweise in einem geeigneten Lösungs- oder Verdünnungsmittel, hergestellt werden.

Die Verbindungen der Formel Ia sind in freier Form nicht beständig, sondern werden in Form ihrer Säureadditionssalze isoliert. Bei der Synthese der Verbindungen der Formel Ia fallen normalerweise die Säureadditionssalze an.

Die benötigten Ausgangsverbindungen der allgemeinen Formel II können in an sich bekannter Weise nach der Strecker'schen Aminonitrilsynthese aus Verbindungen der allgemeinen Formel IV oder ihren Säureadditionssalzen

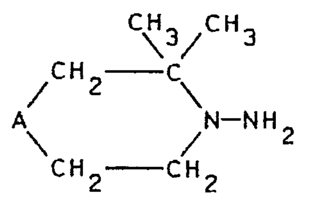

(IV)

worin A die bereits genannte Bedeutung besitzt, durch Umsetzung mit Formaldehyd und Blausäure bzw. Natriumcyanid in einem geeigneten Lösungs- oder Dispergiermittel oder Lösungs- oder Dispergiermittelgemisch, z.B. in Wasser, hergestellt werden, wobei zunächst eine Verbindung der allgemeinen Formel V

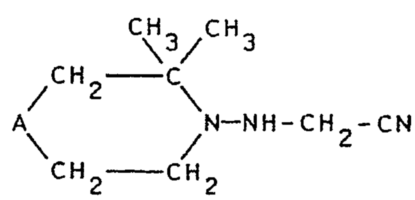

(V)

entsteht, die durch Nitrosierung in die Verbindung II überführt wird. Die Nitrosierung wird in bekannter Weise in einem geeigneten Lösungs- oder Dispergiermittel oder Lösungs- oder Dispergiermittelgemisch, z.B. in Wasser, z.B. bei Temperaturen von -10 bis 10°C durchgeführt. Die salpetrige Säure wird dabei normalerweise aus einem Alkalimetallnitrit, z.B. Natriumnitrit, und Salzsäure erzeugt. Es ist zweckmäßig, die wäßrige Lösung der Verbindung V mit Salzsäure auf einen pH-Wert von 1 bis 3 einzustellen und das Alkalimetallnitrit in Form einer wäßrigen Lösung zu der gerührten und abgekühlten Lösung der Verbindung zuzutropfen. Die Lösung der dabei erhaltenen Verbindung II kann direkt der Cyclisierungsreaktion unterworfen werden. Normalerweise ist es jedoch angebracht, die Nitrosoverbindung II zunächst in einem geeigneten organischen Lösungsmittel aufzunehmen und in ihm, gegebenenfalls nach Zusatz eines weiteren Lösungsmittels, die Cyclisierung zu der Verbindung der Formel Ia durchzuführen.

Die Verbindungen der allgemeinen Formel IV lassen sich, ausgehend von Verbindungen der allgemeinen Formel VI

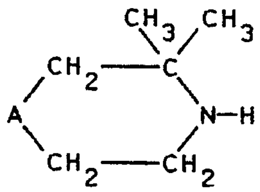

(VI)

dadurch herstellen, daß entweder

a) eine Verbindung der Formel VI zur N-Nitrosoverbindung VII nitrosiert und anschließend, zweckmäßigerweise mit Lithiumaluminiumhydrid, reduziert wird:

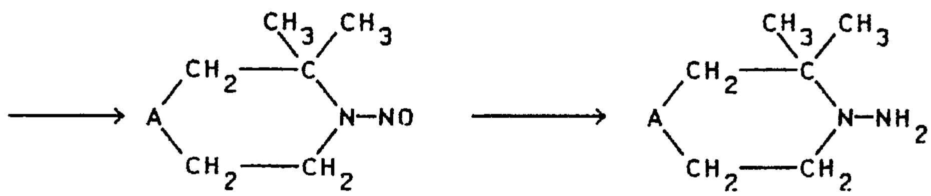

(VI) (VII) (IV)

oder daß in an sich bekannter Weise

b) eine Verbindung der Formel VI mit Kaliumcyanat im sauren Medium in das Harnstoffderivat VIII überführt wird, das dann durch Oxydation mit Natriumhypochlorit nach dem Hoffmann-Abbau in die Verbindung IV überführt wird:

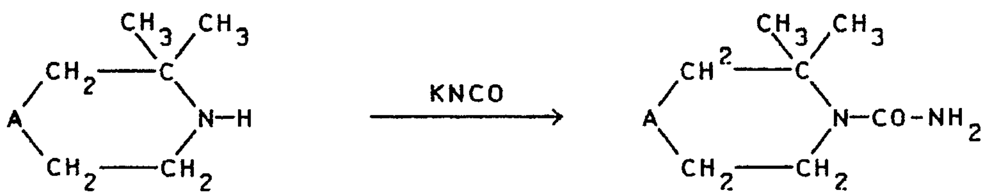


(VI) (VIII)

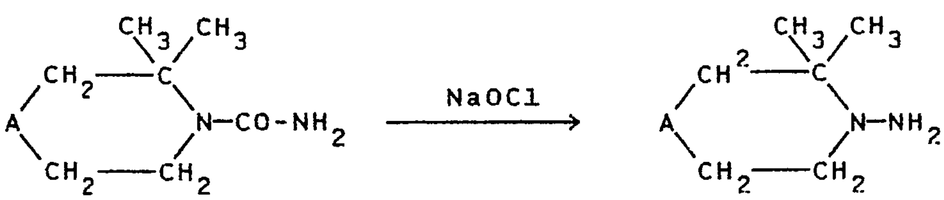


(VIII) (IV)

Erfindungegemäße Verbindungen der Formel I können auch dadurch hergestellt werden, daß eine Verbindung der Formel I mit A = -S- oder ein Säureadditionssalz einer solchen Verbindung, mit einer Sauerstoff abgebenden Substanz umgesetzt und die erhaltene Verbindung gegebenenfalls in ein Säureadditionssalz überführt und isoliert wird, oder daß für den Fall, daß eine Verbindung der Formel Ia erhalten worden ist und eine Verbindung der Formel I mit A = -SO- und $R^2$ = -$COR^2$ hergestellt werden soll, die Verbindung Ia oder ein Säureadditionssalz davon mit einem Acylierungsmittel, das den Rest -$COR^2$ einführt, acyliert wird und die so erhaltene Verbindung isoliert oder gegebenenfalls in ein pharmakologisch annehmbares Säureadditionssalz überführt und dieses isoliert wird.

Als geeignete Sauerstoff abgebende Substanzen sind beispielsweise zu nennen: Wasserstoffperoxid, Peroxosäuren, wie z.B. Perameisen-, Peressig-, Perbenzoe-, m-Chlorperbenzoe-, Perphthal-säure, sowie

Diacylperoxide. Auch Gemische verschiedener Sauerstoff abgebender Substanzen können verwendet werden. Die Sauerstoff abgebende Substanz wird normalerweise in der stöchiometrisch erforderlichen Menge oder in einem geringen Überschuß zu dieser stöchiometrisch erforderlichen Menge eingesetzt. Größere Überschüsse werden vermieden, da sich sonst das Sulfon, d.h. eine Verbindung der Formel I mit A = $-SO_2-$ bildet. Die Umsetzung kann zweckmäßigerweise in einem geeigneten Lösungs- oder Dispergiermittel oder Lösungs- oder Dispermittelgemisch z.B. bei Temperaturen von 0°C, vorzugsweise von Raumtemperatur, bis zum Siedepunkt des Lösungs- oder Dispergiermittels oder Lösungs- oder Dispergiermittelgemischs, durchgeführt werden. Geeignete Lösungs- oder Dispergiermittel sind z.B. die bereits genannten Ether, aliphatische, cycloaliphatische und aromatische Kohlenwasserstoffe, halogenierte aliphatische und aromatische Kohlenwasserstoffe, Wasser, insbesondere aber niedere Carbonsäuren, wie Ameisen- oder Essigsäure.

Eine nach der Umsetzumg mit dem Sauerstoff abgebenden Mittel gewünschtenfalls durchzuführende Acylierung mit einem den Rest $-COR^2$ einführenden Acylierungsmittel wird in der bereits genannten Art und Weise mit einem Acylierungsmittel der Formel III durchgeführt.

Die gewünschtenfalls vorzunehmende Überführung in das Säureadditionssalz wird, wie bereits vorstehend angegeben, in an sich bekannter Weise durchgeführt.

Die Verbindungen der Formel I mit A = -S- können analog den Verbindungen der Formel I mit A = -SO- aus Verbindungen der Formel II mit A = -S- hergestellt werden.

Die erfindungsgemäßen Verbindungen der allgemeinen Formel I und ihre pharmakologisch annehmbaren Säureadditionssalze besitzen wertvolle pharmakologische z.B. hämodynamische, thrombozytenfunktionshemmende und antithrombotische, insbesondere coronar-antithrombotische Eigenschaften. Besonders ausgeprägt ist ihre Wirkung auf das Herz-Kreislauf-System. Verglichen mit bekannten Sydnoniminverbindungen, z.B. solchen der FR-PS-1 496 056 und den in den Beispielen der DE-OS 38 20 210 beschriebenen Verbindungen, besitzen sie überraschenderweise eine erstaunlich längere Wirkungsdauer. Sie senken beispielsweise den Blutdruck ebenso wie den Pulmonalarteriendruck und den linksventrikulären enddiastolischen Druck und tragen so zu einer Entlastung der Herztätigkeit im Sinne einer antianginösen Wirkung bei, ohne dabei eine reflektorische Tachykardie zu provozieren.

Die erfindungsgemäßen Verbindungen der Formel I und ihre pharmakologisch annehmbaren Säureadditionssalze können daher am Menschen als Heilmittel für sich allein, in Mischungen untereinander oder in Form von pharmazeutischen Zubereitungen verabreicht werden, die eine enterale oder parenterale Anwendung gestatten und die als aktiven Bestandteil eine wirksame Dosis mindestens einer erfindungsgemäßen Verbindung der Formel I oder eines Säureadditionssalzes davon, neben einem oder mehreren üblichen pharmazeutisch einwandfreien Träger-oder Verdünnungs- und gegebenenfalls einem oder mehreren Zusatzstoffen enthalten.

Die Heilmittel können oral, z.B. in Form von Tabletten, Filmtabletten, Dragees, Hart- und Weichgelatinekapseln, Mikrokapseln, Granulaten, Pulvern, Pellets, Lösungen, Sirupen, Emulsionen, Suspensionen, Aerosolen, Schäumen, Pillen oder Pastillen verabreicht werden. Die Verabreichung kann aber auch rektal, z.B. in Form von Suppositorien, oder parenteral, z.B. in Form von Injektionslösungen, oder perkutan, z.B. in Form von Salben, Cremes, Gelen, Pasten, Aerosolen, Schäumen, Pudern, Tinkturen, Linimenten oder sog. Transdermalen Therapeutischen Systemen (TTS) erfolgen.

Die pharmazeutischen Präparate können in an sich bekannter Weise unter Verwendung pharmazeutisch inerter anorganischer oder organischer Hilfs-, Träger-, Füll- oder Verdünnungsstoffe hergestellt werden. Für die Herstellung von Pillen, Tabletten, Filmtabletten, Dragees und die Pellet- oder Granulatfüllungen von Hartgelatinekapseln kann man z.B. Calciumphosphate, Lactose, Sorbitol, Mannitol, Stärken, präparierte Stärken, chemisch modifizierte Stärken, Stärkehydrolysate, Cellulose, Cellulosederivate, synthetische Polymere, Talk etc. verwenden. Träger- oder Verdünnungsstoffe für Weichgelatinekapseln und Suppositorien sind z.B. Fette, Wachse, halbfeste und flüssige Polyole, natürliche oder gehärtete Öle etc. Als Träger- oder Verdünnungsstoffe für die Herstellung von Lösungen und Sirupen eignen sich z.B. Wasser, Polyole, Lösungen von Saccharose, Invertzucker, Glukose etc. Als Trägerstoffe für die Herstellung von Injektionslösungen eignen sich z.B. Wasser, Alkohole, Glyzerol, Polyole oder pflanzliche Öle. Als Träger- oder Verdünnungsstoffe für Salben, Cremes und Pasten eignen sich z.B. Naturvaseline, Kunstvaseline, dick- und dünnflüssige Paraffine, Fette, natürliche oder gehärtete pflanzliche und tierische Öle, Neutralöle, Wachse, Wachsalkohole, Polyethylenglykole, Polyacrylsäure, Silicongele etc.

Die pharmazeutischen Präparate können in an sich bekannter Weise neben den Wirk- und Verdünnungs-, Füll- oder Trägerstoffen auch noch einen oder mehrere Zusatzstoffe oder Hilfsmittel, wie z.B. Spreng-, Binde-, Gleit-, Schmier-, Formtrenn-, Netz-, Stabilisierungs-, Emulgier-, Konservierungs-, Süß-, Färbe-, Geschmacks- oder Aromatisierungs-Mittel, Puffersubstanzen, ferner Lösungsmittel oder Lösungsvermittler, Lösungsbeschleuniger, Antischaummittel, Salzbildner, Gelbildner, Verdickungsmittel, Fließregulierungsmittel, Sorptionsmittel, Mittel zur Erzielung eines Depoteffekts oder Mittel, insbesondere Salze, zur Veränderung des osmotischen Drucks, Überzugsmittel oder Antioxidantien etc. enthalten. Sie können auch zwei oder mehrere Verbindungen der Formel I oder ihrer pharmakologisch annehmbaren Säureadditionssalze und noch einen oder mehrere andere therapeutisch wirksame Stoffe enthalten.

Derartige andere therapeutisch wirksame Substanzen können beispielsweise sein: β-Rezeptorenblocker, wie z.B. Propranolol, Pindolol, Metoprolol; Vasodilatatoren, wie z.B. Carbochromen; Beruhigungsmittel, wie z.B. Barbitursäurederivate, 1,4-Benzodiazepine und Meprobamat; Diuretica, wie z.B. Chlorothiazid; das Herz tonisierende Mittel, wie z.B. Digitalispräparate; blutdrucksenkende Mittel, wie z.B. Hydralazin, Dihydralazin, Prazosin, Clonidin, Rauwolfia-Alkaloide; Mittel, die den Fettsäurespiegel im Blut senken, wie z.B. Bezafibrat, Fenofibrat; Mittel für die Thromboseprophylaxe, wie z.B. Phenprocoumon.

In den pharmazeutischen Präparaten kann der Gehalt an dem Wirkstoff oder den Wirkstoffen der Formel I in weiten Grenzen schwanken und z.B. 0,05 bis 50 Gew.%, vorzugsweise 0,05 bis 20 Gew.%, betragen. In festen Darreichungsformen, wie Dragees, Tabletten etc. beträgt der Gehalt an einem oder mehreren Wirkstoffen der Formel I in vielen Fällen 2 bis 20 Gew.%. Flüssige Darreichungsformen, wie Tropfen, Emulsionen und Injektionslösungen enthalten häufig 0,05 bis 2 Gew.%, vorzugsweise 0,05 bis 1 Gew.%, eines oder mehrerer Wirkstoffe der Formel I. Der Gehalt an einem oder mehreren Wirkstoffen der Formel I kann gegebenenfalls in den pharmazeutischen Präparaten teilweise, z.B. bis zu 50 Gew.%, vorzugsweise zu 5 bis 40 Gew.%, durch eine oder mehrere andere therapeutisch wirksame Substanzen ersetzt sein.

Die erfindungsgemäßen Verbindungen der Formel I, ihre pharmakologisch annehmbaren Säureadditionsssalze und pharmazeutische Präparate, welche die erfindungsgemäßen Verbindungen der Formel I oder ihre pharmakologisch annehmbaren Säureadditionssalze als Wirkstoffe enthalten, können am Menschen bei der Bekämpfung bzw. Vorbeugung von Erkrankungen des kardiovaskulären Systems verwendet werden, beispielsweise als antihypertensive Heilmittel bei den verschiedenen Formen des Bluthochdrucks, bei der Bekämpfung bzw. Vorbeugung von Angina pectoris usw. Die Dosierung kann innerhalb weiter Grenzen variieren und ist in jedem einzelnen Fall den individuellen Gegebenheiten anzupassen. Im allgemeinen ist bei oraler Verabreichung pro menschlichem Individuum eine Tagesdosis von etwa 0,5 bis 100 mg, vorzugsweise 1 bis 20 mg, angemessen. Auch bei anderen Applikationsformen liegt die Tagesdosis, wegen der guten Resorption der Wirkstoffe, in ähnlichen Mengenbereichen, d.h. im allgemeinen ebenfalls bei 0,5 bis 100 mg/Mensch. Die Tagesdosis wird normalerweise in mehrere, z.B. 2 bis 4 Teilverabreichungen aufgeteilt.

Die pharmakologische Wirkung der erfindungsgemäßen Verbindungen der Formel I wurde nach einer modifizierten Methode von Godfraind and Kaba (Arch.Int. Pharmacodyn. Ther. 196, (Suppl) 35 bis 49, 1972) und von Schüman et al (Naunyn-Schmiedeberg's Arch. Pharmacol. 289, 409 bis 418, 1975) ermittelt. Dabei werden Spiralstreifen der Arteria pulmonalis des Meerschweinchens nach Äquilibrierung in calciumfreier Tyrodelösung mit 40 mmol/l Kalium depolarisiert. Ein Zusatz von 0,5 mmol/l $CaCl_2$ löst dann eine Kontraktion aus.

Die relaxierende Wirkung der Prüfsubstanz wird durch kumulative Zugabe in 1/2 log 10 abgestuften Konzentrationen ermittelt. Aus der Konzentrationswirkungskurve (Abszisse: -log mol/l Prüfsubstanz, Ordinate: % Hemmung der maximalen Kontraktion, Mittelwert von 4 bis 6 Gefäßstreifen) wird die Konzentration der Prüfsubstanz ermittelt, welche die Kontraktion um 50 % hemmt (= $IC_{50}$, mol/l). Die Wirkungsdauer der Prüfsubstanz ergibt sich aus der Zeit, die nach der Zugabe der Prüfsubstanz benötigt wird, bis der Ausgangswert wieder erreicht ist. In der folgenden Tabelle sind die so erhaltenen Werte angegeben.

$IC_{50}$-Werte und Wirkungsdauer für Sydnonimine der Formel

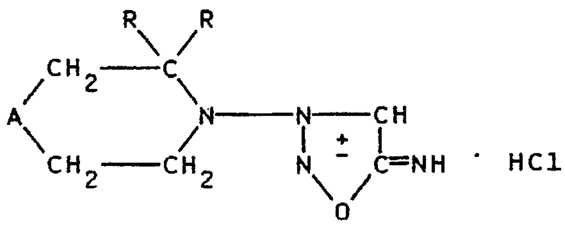

| Verbindung Nr. | A | R | $IC_{50}$ in mol/l | Wirkungsdauer in min |
|---|---|---|---|---|
| 1 | 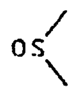 | $CH_3$ | $3 \cdot 10^{-6}$ | 270 |
| 2 | 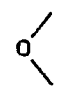 | H | $1 \cdot 10^{-6}$ | 80 |
| 3 | 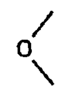 | $CH_3$ | $1 \cdot 10^{-6}$ | 120 |

Bei der Verbindung Nr. 1 der Tabelle handelt es sich um die erfindungsgemäße Verbindung 3-(3,3-Dimethyl-perhydro-1-oxo-1,4-thiazin-4-yl)-sydnonimin-hydrochlorid.

Bei der Verbindung Nr. 2 der Tabelle handelt es sich um die aus Beispiel 2 der FR-PS-1 406 056 bekannte Verbindung 3-Morpholino-sydnonimin-hydrochlorid.

Bei der Verbindung Nr. 3 der Tabelle handelt es sich um die Verbindung 3-(3,3-Dimethylmorpholin-4-yl)-sydnoniminhydrochlorid des Beispiels Nr. 1 der DE-OS 38 20 210.

Wie aus der vorstehenden Tabelle zu ersehen ist, besitzt die erfindungsgemäße Verbindung bei vergleichbarem $IC_{50}$-Wert eine wesentlich längere Wirkungsdauer als die beiden Vergleichsverbindungen.

Soweit in den nachfolgenden Beispielen nichts anderes angegeben ist, handelt es sich bei Prozentangaben um Gewichtsprozente.

Beispiel 1

3-(3,3-Dimethyl-perhydro-1-oxo-1,4-thiazin-4-yl)-sydnonimin-hydrochlorid

a) 2,2-Dimethylaziridin

100 g 2-Amino-2-methyl-propan-1-ol werden in 200 ml Wasser gelöst. Dann wird eine kalte Lösung von 110 g konz. Schwefelsäure in 200 ml Wasser unter Rühren zugetropft und danach das Wasser bei Normaldruck abdestilliert, bis eine Innentemperatur von 115°C erreicht ist. Dann wird im Wasserstrahlvakuum bis zu einer Badtemperatur von 180°C weiter destilliert und der Kolbeninhalt unter diesen Bedingungen so lange gehalten, bis er fest wird. Anschließend wird bei Wasserstrahlvakuum und 180°C noch 1 Stunde weiter erhitzt.

Das Produkt wird mit einer Lösung von 100 g NaOH in 200 ml Wasser versetzt, zerkleinert und über Nacht stehengelassen. Von der Suspension wird unter Normaldruck Wasser abdestilliert. Zu dem Rückstand werden 50 g KOH unter Rühren zugegeben und 3 Stunden gerührt. Nach Stehenlassen wird die obere Schicht abgetrennt, mit 20 g KOH versetzt und über Nacht stehengelassen. Das öl wird abgegossen, mit 10 g KOH versetzt und unter Normaldruck destilliert.

Ausbeute: 60,3 g

b) (2-Hydroxy-ethyl)-(2-amino-2-methyl-propyl)-sulfid

234 g 2-Mercaptoethanol werden in 90 ml Dimethoxyethan gelöst. Zu dieser Lösung werden 63,3 g 2,2-Dimethylaziridin zugetropft und 4 Stunden bei 70°C gerührt, eingeengt und im Wasserstrahlvakuum destilliert.

Kp.: 141-142°C (bei 20 mbar) Fp.: 49-51°C

c) 3,3-Dimethyl-perhydro-1,4-thiazin

Zu einer Lösung von 14,9 g (2-Hydroxy-ethyl)-(2-amino-2-methyl-propyl)-sulfid (Stufe b) in 100 ml Dimethoxyethan wird unter Rühren bis zur Sättigung Chlorwasserstoff eingeleitet und dann 17,9 g Thionylchlorid zugetropft und anschließend 1 Stunde bei Raumtemperatur gerührt und danach 1 Stunde am Rückfluß erhitzt. Die Lösung wird dann im Wasserstrahlvakuum eingeengt. Der Rückstand (2-Chloroethyl)-(2-amino-2-methyl-propyl)-sulfid-hydrochlorid kristallisiert aus.

Das erhaltene Hydrochlorid wird in Wasser gelöst, mit Pottasche stark alkalisch gestellt, in 100 ml Toluol aufgenommen und die wäßrige Phase nochmals mit 2 x 30 ml Toluol ausgeschüttelt. Die organischen Phasen werden vereinigt, über Pottasche getrocknet, eingeengt und im Wasserstrahlvakuum destilliert.

Ausbeute: 8,1 g Kp.: 73-75°C (bei 20 mbar)

d) 4-Nitroso-3,3-dimethyl-perhydro-1,4-thiazin

Eine Mischung von 15,7 g 3,3-Dimethyl-perhydro-1,4-thiazin (Stufe c) und 60 ml Eiswasser werden unter Kühlung mit 12 ml 10 n HCl versetzt. Dann wird bei 4 bis 6°C eine Lösung von 12,4 g Natriumnitrit in 40 ml Wasser zugetropft. Man rührt bei Raumtemperatur 4 Stunden nach, stellt mit Pottasche alkalisch und schüttelt mit Ethylacetat aus. Die organische Phase wird getrocknet und im Wasserstrahlvakuum eingeengt.

Ausbeute: 17,5 g Fp.: Gelbes öl

e) 4-Amino-3,3-dimethyl-perhydro-1,4-thiazin-hydrochlorid

Eine Lösung von 17,2 g 4-Nitroso-3,3-dimethyl-perhydro-1,4-thiazin (Stufe d) in 150 ml Tetrahydrofuran wird bei 60-65°C portionsweise mit 4,4 g Lithiumaluminiumhydrid versetzt und danach 2 Stunden am Rückfluß erhitzt. Die Suspension wird abgekühlt und eine Lösung von 5 ml Wasser in 50 ml Tetrahydrofuran zugetropft. Nach 20 Minuten werden 10 ml 27gew.%ige Natronlauge zugetropft und einige Stunden stehen gelassen. Dann wird abfiltriert und im eisgekühlten Filtrat das Hydrochlorid gefällt.

Ausbeute: 12,2 g

f) 4-(2-Cyanoethyl-amino)-3,3-dimethyl-perhydro-1,4-thiazin

Zu einer auf -3 bis -5°C gekühlten Lösung von 12,1 g 4-Amino-3,3-dimethyl-perhydro-1,4-thiazin-hydrochlorid (Stufe e) wird eine Lösung von 6,1 g KCN in 30 ml Wasser zugetropft. Dann wird mit Salzsäure der pH Wert der Lösung auf 7,2 gestellt und danach 16,3 g 39gew.%iges wäßriges Formaldehyd zugesetzt, wobei der pH Wert auf 6 bis 7 gehalten wird. Es wird über Nacht bei auf Raumtemperatur ansteigende Temperatur nachgerührt. Die Suspension wird mit Ethylacetat ausgeschüttelt, die organische Phase getrocknet und eingeengt.

Ausbeute: 9,4 g Fp.: Öl

g) 3-(3,3-Dimethyl-perhydro-1,4-thiazin-4-yl)-sydnoniminhydrochlorid

9,4 g 4-(2-Cyanoethyl-amino)-3,3-dimethyl-perhydro-1,4-thiazin (Stufe f), 40 ml Ethylacetat, 40 g Eiswasser und 5 ml 10 n Salzsäure werden auf -5°C gekühlt. In diese Mischung werden 5,3 g Natriumnitrit eingetragen und anschließend 3 Stunden bei Raumtemperatur gerührt. Die organische Phase wird abgetrennt, getrocknet, abfiltriert und unter Kühlen 20 ml einer gesättigten Lösung von HCl in Isopropanol zugetropft. Es wird über Nacht bei Raumtemperatur nachgerührt. Der Niederschlag wird abgetrennt und aus Isopropanol umkristallisiert.

Ausbeute: 5,9 g Fp.: 172°C (Zersetzung)

h) 3-(3,3-Dimethyl-perhydro-1-oxo-1,4-thiazin-4-yl)-sydnonimin-hydrochlorid

Die Lösung von 3 g 3-(3,3-Dimethyl-perhydro-1,4-thiazin-4-yl)-sydnonimin-hydrochlorid (Stufe g) wird bei Raumtemperatur unter Rühren mit 1,2 g einer 35%igen wäßrigen Wasserstoffperoxidlösung versetzt, wobei die Temperatur etwas ansteigt. Die Mischung wird 2 Stunden gerührt und dann im Wasserstrahlvakuum eingeengt und der Rückstand aus Essigsäureethylester umkristallsiert.

Ausbeute: 2,5 g Fp.: 192°C (Zers.)

Beispiel 2

N-p-Anisoyl-3-(3,3-dimethylperhydro-1-oxo-1,4-thiazin-4-yl)-sydnonimin

3 g 3-(3,3-Dimethyl-perhydro-1-oxo-1,4-thiazin-4-yl)-sydnonimin-hydrochlorid (Beispiel 1 h) werden in 20 ml Eiswasser gelöst und 2 g $NaHCO_3$ zugegeben. Dann werden 2,1 g p-Anisoylchlorid, gelöst in 30 ml Ethylacetat, zugetropft. Es wird 3 Stunden nachgerührt, wobei die Temperatur bis auf Raumtemperatur ansteigt. Dann werden die Phasen getrennt, die wäßrige Phase mit Ethylacetat ausgeschüttelt, die organischen Phasen vereinigt, getrocknet und eingeengt. Das erhaltene kristalline Produkt wird aus

Isopropanol umkristallisiert.
Ausbeute: 1,8 g Fp.: 183 bis 185°C (Zersetzung)

Beispiel 3

N-Ethoxycarbonyl-3-(3,3-dimethyl-perhydro-1-oxo-1,4-thiazin-4-yl)-sydnonimin

Die Verbindung wird analog Beispiel 2 mit Chlorameisensäureethylester (1,8 g) anstelle von p-Anisoylchlorid erhalten und aus Essigsäureisopropylester umkristallisiert.
Ausbeute: 1,4 g Fp.: 138°C (Zersetzung)

Beispiel 4

N-Pivaloyl-3-(3,3-dimethyl-perhydro-1-oxo-1,4-thiazin-4-yl)-sydnonimin

Die Verbindung wird analog Beispiel 2 mit 2,1 g Pivalinsäurechlorid anstelle von p-Anisoylchlorid erhalten und aus Diisopropylether umkristallisiert.
Ausbeute: 1,3 g Fp.: 110°C (Zersetzung)

In den nachfolgenden Beispielen A bis H werden pharmazeutische Präparate beschrieben.

Beispiel A

Gelatineweichkapseln, enthaltend 5 mg Wirkstoff pro Kapsel:

| | pro Kapsel |
|---|---|
| Wirkstoff | 5 mg |
| Aus Kokosfett fraktioniertes Triglycerid-Gemisch | 150 mg |
| Kapselinhalt | 155 mg |

Beispiel B

Injektionslösung, enthaltend 1 mg Wirkstoff pro ml:

| | pro ml |
|---|---|
| Wirkstoff | 1,0 mg |
| Polyethylenglycol 400 | 0,3 ml |
| Natriumchlorid | 2,7 mg |
| Wasser zu Injektionszwecken | ad 1 ml |

Beispiel C

Emulsion, enthaltend 3 mg Wirkstoff pro 5 ml

| | pro 100 ml Emulsion |
|---|---|
| Wirkstoff | 0,06 g |
| Neutralöl | q.s. |
| Natriumcarboxymethylcellulose | 0,6 g |
| Polyoxyethylen-stearat | q.s. |
| Glycerin rein | 0,2 bis 2,0 g |
| Geschmacksstoff | q.s. |
| Wasser (entsalzt oder destilliert) | ad 100 ml |

Beispiel D

Rektale Arzneiform, enthaltend 4 mg Wirkstoff pro Suppositorium

| | pro Suppositorium |
|---|---|
| Wirkstoff | 4 mg |
| Suppositoriengrundmasse | ad 2 g |

**Beispiel E**

Tabletten, enthaltend 2 mg Wirkstoff pro Tablette

| | pro Tablette |
|---|---|
| Wirkstoff | 2 mg |
| Lactose | 60 mg |
| Maisstärke | 30 mg |
| lösliche Stärke | 4 mg |
| Magnesiumstearat | 4 mg |
| | 100 mg |

**Beispiel F**

Dragees, enthaltend 1 mg Wirkstoff pro Dragee

| | pro Dragee |
|---|---|
| Wirkstoff | 1 mg |
| Maisstärke | 100 mg |
| Lactose | 60 mg |
| sec Calciumphosphat | 30 mg |
| lösliche Stärke | 3 mg |
| Magnesiumstearat | 2 mg |
| kolloidale Kieselsäure | 4 mg |
| | 200 mg |

**Beispiel G**

Für die Herstellung des Inhalts von Hartgelatinekapseln eignen sich die folgenden Rezepturen:

| | |
|---|---|
| a) Wirkstoff | 10 mg |
| Maisstärke | 190 mg |
| | 200 mg |

| | |
|---|---|
| b) Wirkstoff | 40 mg |
| Milchzucker | 80 mg |
| Maisstärke | 80 mg |
| | 200 mg |

Beispiel H

Tropfen können nach folgender Rezeptur hergestellt werden (20 mg Wirkstoff in 1 ml = 20 Tropfen):

| | |
|---|---|
| Wirkstoff | 2,00 g |
| Benzoesäuremethylester | 0,07 g |
| Benzoesäureethylester | 0,03 g |
| Ethanol 96%ig | 4 ml |
| entmineralisiertes Wasser ad | 100 ml |

**Patentansprüche**

1. Substituierte 3-Aminosydnonimine der allgemeinen Formel I

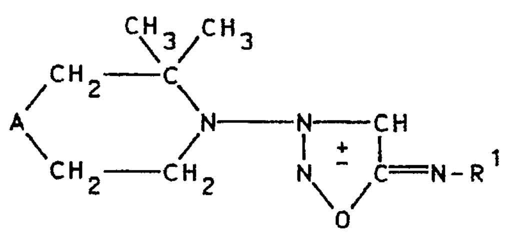

(I)

und ihre pharmakologisch annehmbaren Säureadditionssalze, worin
A den Rest -SO-,
$R^1$ den Rest -CO-$R^2$ oder Wasserstoff,
$R^2$ ($C_1$ bis $C_4$)Alkyl, ($C_1$ bis $C_4$)Alkoxy-($C_1$ bis $C_4$)alkyl, ($C_1$ bis $C_4$)Alkoxy, ($C_5$ bis $C_7$)Cycloalkyl, Phenyl, einen mit 1 bis 3 Halogenatomen und/oder 1 bis 3 Alkylresten mit 1 bis 4 C-Atomen und/oder 1 bis 3 Alkoxyresten mit 1 bis 4 C-Atomen mono-, di- oder trisubstituierten Phenylrest bedeuten.

2. Substituierte 3-Aminosydnonimine nach Anspruch 1, dadurch gekennzeichnet, daß $R^2$ ($C_1$ bis $C_4$)Alkyl, ($C_1$ bis $C_4$)Alkoxy, Phenyl, oder durch Chlor, Methyl oder Methoxy monosubstituiertes Phenyl bedeutet.

3. Die pharmakologisch annehmbaren Säureadditionssalze, insbesondere das Hydrochlorid der Verbindung 3-(3,3-Dimethyl-perhydro-1-oxo-1,4-thiazin-4-yl)-sydnonimin.

4. N-p-Anisoyl-3-(3,3-dimethyl-perhydro-1-oxo-1,4-thiazin-4-yl)-sydnonimin und seine pharmakologisch annehmbaren Säureadditionssalze, insbesondere sein Hydrochlorid.

5. N-Ethoxycarbonyl-3-(3,3-dimethyl-perhydro-1-oxo-1,4-thiazin-4-yl)-sydnonimin und seine pharmakologisch annehmbaren Säureadditionssalze, insbesondere sein Hydrochlorid.

6. N-Pivaloyl-3-(3,3-dimethyl-perhydro-1-oxo-1,4-thiazin-4-yl)-sydnonimin und seine pharmakologisch annehmbaren Säureadditionssalze, insbesondere sein Hydrochlorid.

7. Verfahren zur Herstellung der in Anspruch 1 und/oder 2 angegebenen Verbindungen der Formel I und/oder der in einem oder mehreren der Ansprüche 3 bis 6 angegebenen Verbindungen, dadurch gekennzeichnet, daß eine Verbindung der allgemeinen Formel II

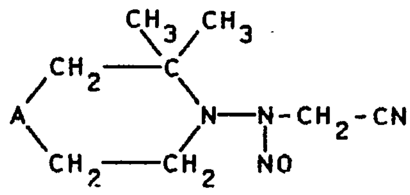

(II)

worin A den Rest -SO- bedeutet, zu einer Verbindung der Formel Ia

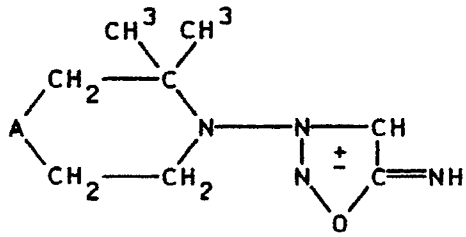

(Ia)

cyclisiert und die erhaltene Verbindung als Säureadditionssalz isoliert wird, und daß für den Fall, daß eine Verbindung der Formel I mit $R^1$ = $-COR^2$ hergestellt werden soll, die Verbindung Ia oder ein Säureadditionssalz davon mit einem Acylierungsmittel, das den Rest $-COR^2$ einführt, acyliert wird und die so erhaltene Verbindung isoliert oder gegebenenfalls in ein pharmakologisch annehmbares Säureadditionssalz überführt, und dieses isoliert wird.

**8.** Verfahren nach Anspruch 7, dadurch gekennzeichnet, daß die Cyclisierung in einem Lösungs-, Dispergier oder Verdünnungsmittel bei Temperaturen von -10 bis 40°C, vorzugsweise 0 bis 20°C, mit Hilfe eines Cyclisierungsmittels, das in wäßriger Lösung einen pH-Wert unter 3 einstellt, durchgeführt wird.

**9.** Verfahren zur Herstellung der in Anspruch 1 und/oder 2 angegebenen Verbindungen der Formel I und/oder der in einem oder mehreren der Ansprüche 3 bis 6 angegebenen Verbindungen, dadurch gekennzeichnet, daß eine Verbindung der allgemeinen Formel Ib

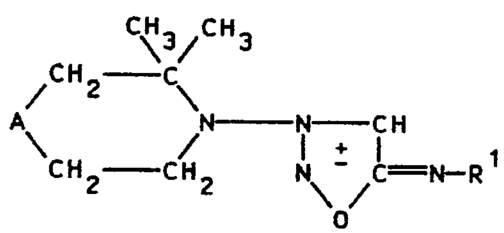

(Ib)

worin
A den Rest -S- und
$R^1$ Wasserstoff oder den Rest $-COR^2$,
$R^2$ ($C_1$ bis $C_4$)Alkyl, ($C_1$ bis $C_4$)Alkoxy-($C_1$ bis $C_4$)alkyl, ($C_1$ bis $C_4$)Alkoxy, ($C_5$ bis $C_7$)-Cycloalkyl, Phenyl, einen mit 1 bis 3 Halogenatomen und/oder 1 bis 3 Alkylresten mit 1 bis 4 C-Atomen und/oder 1 bis 3 Alkoxyresten mit 1 bis 4 C-Atomen mono-, di- oder trisubstituierten Phenylrest bedeuten, oder ein Säureadditionssalz davon mit einer Sauerstoff abgebenden Verbindung umgesetzt und die erhaltene Verbindung gegebenenfalls in ein Säureadditionssalz überführt und isoliert wird, oder daß für den Fall, daß eine Verbindung der Formel I mit A = -SO- und $R^1$ = H erhalten worden ist und eine Verbindung der Formel I mit A = -SO-und $R^2$ = $-COR^2$ hergestellt werden soll, die erhaltene Verbindung oder ein Säureadditionssalz davon mit einem Acylierungsmittel, das den Rest $-COR^2$ einführt, acyliert wird und die so erhaltene Verbindung isoliert oder gegebenenfalls in ein pharmakologisch annehmbares Säureadditionssalz überführt und dieses isoliert wird.

**10.** Verfahren zur Herstellung substituierter 3-Aminosydnonimine der allgemeinen Formel I

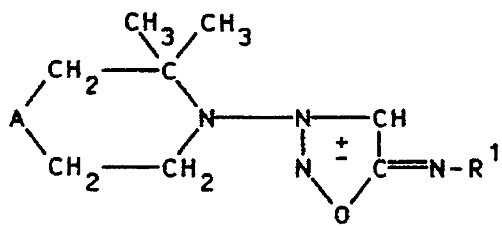

(I)

und ihrer pharmakologisch annehmbaren Säureadditionssalze, worin
A den Rest -SO-,
$R^1$ den Rest -CO-$R^2$,
$R^2$ ($C_1$ bis $C_4$)Alkyl, ($C_1$ bis $C_4$)Alkoxy-($C_1$ bis $C_4$)alkyl, ($C_1$ bis $C_4$)Alkoxy, ($C_5$ bis $C_7$)Cycloalkyl, Phenyl, einen mit 1 bis 3 Halogenatomen und/oder 1 bis 3 Alkylresten mit 1 bis 4 C-Atomen und/oder 1 bis 3 Alkoxyresten mit 1 bis 4 C-Atomen mono-, di- oder trisubstituierten Phenylrest bedeuten, dadurch gekennzeichnet, daß eine Verbindung der Formel I, worin $R^1$ = Wasserstoff bedeutet oder ein Säureadditionssalz davon, mit einem Acylierungsmittel, das den Rest -$COR^2$ einführt, acyliert wird und die so erhaltene Verbindung isoliert oder gegebenenfalls in ein pharmakologisch annehmbares Säureadditionssalz überführt und dieses isoliert wird.

**11.** 3-Amino-sydnonimine und ihre pharmakologisch annehmbaren Säureadditionssalze eines oder mehrerer der Ansprüche 1 bis 5 als pharmakologische Wirkstoffe zur Anwendung bei der Bekämpfung und Vorbeugung cardiovaskulärer Erkrankungen oder als pharmazeutische Wirkstoffe zur Herstellung pharmazeutischer Präparate.

**12.** Pharmazeutisches Präparat, dadurch gekennzeichnet, daß es eine Verbindung einer der Ansprüche 1 bis 6 oder ein Säureadditionssalz davon als Wirkstoff zusammen mit pharmazeutisch annehmbaren Träger- und Zusatzstoffen und gegebenenfalls noch eine oder mehrere andere pharmakologische Wirkstoffe enthält.

**Patentanspruch für folgende Vertragsstaaten: ES und GR**

**1.** Verfahren zur Herstellung substituierter 3-Aminosydnonimine der allgemeinen Formel I

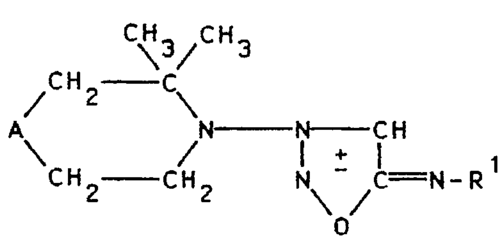

(I)

und ihrer pharmakologisch annehmbaren Säureadditionssalze, worin
A den Rest -SO-,
$R^1$ den Rest -CO-$R^2$ oder Wasserstoff,
$R^2$ ($C_1$ bis $C_4$)Alkyl, ($C_1$ bis $C_4$)Alkoxy-($C_1$ bis $C_4$)alkyl, ($C_1$ bis $C_4$)Alkoxy, ($C_5$ bis $C_7$)Cycloalkyl, Phenyl, einen mit 1 bis 3 Halogenatomen und/oder 1 bis 3 Alkylresten mit 1 bis 4 C-Atomen und/oder 1 bis 3 Alkoxyresten mit 1 bis 4 C-Atomen mono-, di- oder trisubstituierten Phenylrest bedeuten, dadurch gekennzeichnet, daß eine Verbindung der allgemeinen Formel II

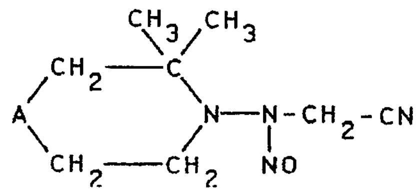

(II)

worin A den Rest -SO- bedeutet, zu einer Verbindung der Formel Ia

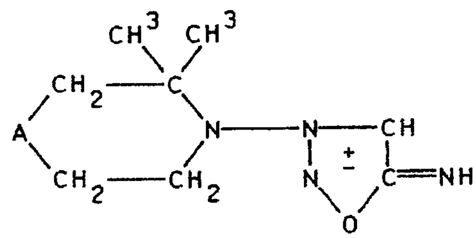

(Ia)

cyclisiert und die erhaltene Verbindung als Säureadditionssalz isoliert wird, und daß für den Fall, daß eine Verbindung der Formel I mit $R^1$ = $-COR^2$ hergestellt werden soll, die Verbindung Ia oder ein Säureadditionssalz davon mit einem Acylierungsmittel, das den Rest $-COR^2$ einführt, acyliert wird und die so erhaltene Verbindung isoliert oder gegebenenfalls in ein pharmakologisch annehmbares Säureadditionssalz überführt, und dieses isoliert wird.

2. Verfahren nach Anspruch 1, dadurch gekennzeichnet, daß die Cyclisierung in einem Lösungs-, Dispergier oder Verdünnungsmittel bei Temperaturen von -10 bis 40°C, vorzugsweise 0 bis 20°C, mit Hilfe eines Cyclisierungsmittels, das in wäßriger Lösung einen pH-Wert unter 3 einstellt, durchgeführt wird.

3. Verfahren nach Anspruch 1 und/oder 2, dadurch gekennzeichnet, daß ein Acylierungsmittel eingesetzt wird, bei dem $R^2$ ($C_1$ bis $C_4$)Alkyl, ($C_1$ bis $C_4$)Alkoxy, Phenyl, oder durch Chlor, Methyl oder Methoxy monosubstituiertes Phenyl bedeutet.

4. Verfahren nach Anspruch 1 und/oder 2, dadurch gekennzeichnet, daß die Ausgangsverbindung der Formel II so ausgewählt wird, daß ein pharmakologisch annehmbares Säureadditionssalz, insbesondere das Hydrochlorid der Verbindung 3-(3,3-Dimethyl-perhydro-1-oxo-1,4-thiazin-4-yl)-sydnonimin entsteht.

5. Verfahren nach einem oder mehreren der Ansprüche 1 bis 3, dadurch gekennzeichnet, daß die Ausgangsverbindungen so ausgewählt werden, daß das N-p-Anisoyl-3-(3,3-dimethyl-perhydro-1-oxo-1,4-thiazin-4-yl)-sydnonimin oder eines seiner pharmakologisch annehmbaren Säureadditionssalze, insbesondere sein Hydrochlorid, entsteht.

6. Verfahren nach einem oder mehreren der Ansprüche 1 bis 3, dadurch gekennzeichnet, daß die Ausgangsverbindungen so ausgewählt werden, daß das N-Ethoxycarbonyl-3-(3,3-dimethyl-perhydro-1-oxo-1,4-thiazin-4-yl)-sydnonimin oder seine pharmakologisch annehmbaren Säureadditionssalze, insbesondere sein Hydrochlorid, entsteht.

7. Verfahren nach einem oder mehreren der Ansprüche 1 bis 3, dadurch gekennzeichnet, daß die Ausgangsverbindungen so ausgewählt werden, daß das N-Pivaloyl-3-(3,3-dimethylperhydro-1-oxo-1,4-thiazin-4-yl)-sydnonimin oder seine pharmakologisch annehmbaren Säureadditionssalze, insbesondere sein Hydrochlorid, entsteht.

8. Verfahren zur Herstellung substituierter 3-Aminosydnonimine der allgemeinen Formel I

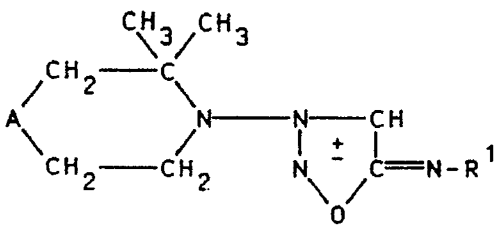

( I )

und ihrer pharmakologisch annehmbaren Säureadditionssalze, worin
A den Rest -SO-,
$R^1$ den Rest -CO-$R^2$,
$R^2$ ($C_1$ bis $C_4$)Alkyl, ($C_1$ bis $C_4$)Alkoxy-($C_1$ bis $C_4$)alkyl, ($C_1$ bis $C_4$)Alkoxy, ($C_5$ bis $C_7$)Cycloalkyl, Phenyl, einen mit 1 bis 3 Halogenatomen und/oder 1 bis 3 Alkylresten mit 1 bis 4 C-Atomen und/oder 1 bis 3 Alkoxyresten mit 1 bis 4 C-Atomen mono-, di- oder trisubstituierten Phenylrest bedeuten, dadurch gekennzeichnet, daß eine Verbindung der Formel I, worin $R^1$ = Wasserstoff bedeutet oder ein Säureadditionssalz davon, mit einem Acylierungsmittel, das den Rest -COR$^2$ einführt, acyliert wird und die so erhaltene Verbindung isoliert oder gegebenenfalls in ein pharmakologisch annehmbares Säureadditionssalz überführt und dieses isoliert wird.

**9.** Verwendung von substituierten 3-Aminosydnoniminen der allgemeinen Formel I

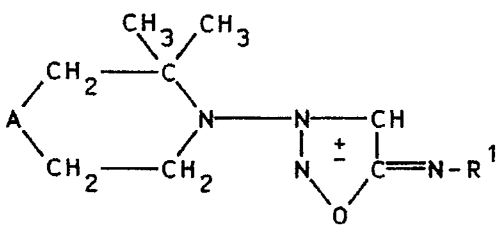

( I )

und ihrer pharmakologisch annehmbaren Säureadditionssalze, worin
A den Rest -SO-,
$R^1$ den Rest -CO-$R^2$ oder Wasserstoff,
$R^2$ ($C_1$ bis $C_4$)Alkyl, ($C_1$ bis $C_4$)Alkoxy-($C_1$ bis $C_4$)alkyl, ($C_1$ bis $C_4$)Alkoxy, ($C_5$ bis $C_7$)Cycloalkyl, Phenyl, einen mit 1 bis 3 Halogenatomen und/oder 1 bis 3 Alkylresten mit 1 bis 4 C-Atomen und/oder 1 bis 3 Alkoxyresten mit 1 bis 4 C-Atomen mono-, di- oder trisubstituierten Phenylrest bedeuten als pharmakologische Wirkstoffe zur Anwendung bei der Bekämpfung und Vorbeugung cardiovaskulärer Erkrankungen oder als pharmazeutische Wirkstoffe zur Herstellung pharmazeutischer Präparate.

**10.** Verfahren zur Herstellung eines pharmazeutischen Präparates, dadurch gekennzeichnet, daß ein substituiertes 3-Aminosydnonimin der allgemeinen Formel I

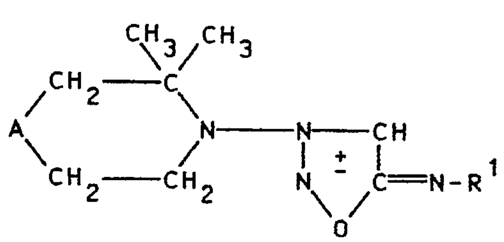

( I )

und/oder ein pharmakologisch annehmbares Säureadditionssalz davon, worin
A den Rest -SO-,
$R^1$ den Rest -CO-$R^2$ oder Wasserstoff,
$R^2$ ($C_1$ bis $C_4$)Alkyl, ($C_1$ bis $C_4$)Alkoxy-($C_1$ bis $C_4$)alkyl, ($C_1$ bis $C_4$)Alkoxy, ($C_5$ bis $C_7$)Cycloalkyl,

Phenyl, einen mit 1 bis 3 Halogenatomen und/oder 1 bis 3 Alkylresten mit 1 bis 4 C-Atomen und/oder 1 bis 3 Alkoxyresten mit 1 bis 4 C-Atomen mono-, di- oder trisubstituierten Phenylrest bedeuten als Wirkstoff zusammen mit pharmazeutisch annehmbaren Träger- und/oder Zusatzstoffen und gegebenenfalls noch ein oder mehrere andere pharmakologische Wirkstoffe in an sich bekannter Weise in eine zur Verabreichung geeignete pharmazeutische Zubereitung überführt werden.

Europäisches Patentamt

# EUROPÄISCHER RECHERCHENBERICHT

Nummer der Anmeldung

EP 91107566.1

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int Cl 5) |
| A | DE - A1 - 2 930 736 (CASSELLA) * Ansprüche 1,13,14 * -- | 1,12 | C 07 D 417/04<br>A 61 K 31/54 |
| A | DE - A1 - 3 702 083 (CASSELLA) * Ansprüche 1,7 * -- | 1,12 | |
| A | EP - B1 - 0 206 219 (CASSELLA) * Anspruch 1; Formel Ia * -- | 1 | |
| A | EP - A2 - 0 312 773 (CASSELLA) * Ansprüche 1,10 * -- | 1,12 | |
| A | EP - A1 - 0 327 808 (CASSELLA) * Ansprüche 1,8 * -- | 1,12 | |
| A | EP - A1 - 0 346 694 (CASSELLA) * Ansprüche 1,12 * ---- | 1,12 | RECHERCHIERTE SACHGEBIETE (Int Cl 5)<br>C 07 D 417/00<br>C 07 D 413/00 |

Der vorliegende Recherchenbericht wurde für alle Patentansprüche erstellt.

| Recherchenort | Abschlußdatum der Recherche | Prüfer |
|---|---|---|
| WIEN | 01-08-1991 | HAMMER |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument